# EUROPEAN PATENT SPECIFICATION

(11) **EP 3 153 196 B1**
(45) Date of publication and mention of the grant of the patent: **11.01.2023**
(21) Application number: 16195290.8
(22) Date of filing: 05.12.2012
(51) Int. Cl.: A61M 5/24, A61M 5/32

(54) **SYRINGE CARRIER**
SPRITZENTRÄGER
SUPPORT DE SERINGUE

(30) Priority: 08.12.2011 EP 11192585
(43) Date of publication of application: 12.04.2017
(62) Divisional of application: 12794996.4
(73) Proprietor: Sanofi-Aventis Deutschland GmbH, 65929 Frankfurt am Main (DE)
(72) Inventor: HOURMAND, Yannick, Haslingfield, Cambridgeshire CB23 1ND (GB); JENNINGS, Douglas Ivan, Royston, Hertfordshire SG8 7XU (GB); EKMAN, Matthew, Macclesfield, Cheshire SK10 1RD (GB)
(74) Representative: Schmidt, Christian

(56) References cited:
- WO-A1-2011/001161
- US-A- 3 144 178
- US-A- 4 931 040
- US-A- 5 078 698
- US-A- 5 451 214

## Description

### Technical Field

The invention relates to syringe carrier.

### Background of the Invention

In a conventional medicament delivery device (e.g., an autoinjector), a pre-filled syringe is housed in a carrier which is axially movable to achieve needle penetration in an injection site and, optionally, needle withdrawal. A conventional carrier provides shoulders that are adapted to engage a neck on the syringe and prevent the syringe from disengaging the carrier. Because syringes are generally supplied with rigid needle shields covering the needle and those needle shields have a diameter greater than a diameter between the shoulders, a separate assembly step is required - inserting the syringe in the carrier and then attaching the rigid needle shield to the needle. Accordingly, there is a need for a syringe carrier which does not require this separate assembly step.

US 4,931,040 A discloses a safety syringe, such as a dental syringe, comprising a hollow cylinder, a disposable, prefilled fluid medication carpule received within the interior of the cylinder, and a needle carrying hub supporting a needle cannula so that the cannula extends axially from the carpule and outwardly of the cylinder, whereby to administer an injection. A piston assembly is movable axially through the carpule to first expulse the medication therefrom and to then engage and retract the needle hub and its associated needle cannula into the carpule, so that the needle will be completely surrounded and shielded by said carpule. A slide lock is connected around one end of the medication carpule and interfaced with the needle carrying hub to control the relocation of the hub and corresponding retraction of the cannula through the carpule. The slide lock is selectively moved relative to the carpule between needle retaining and needle releasing positions to either anchor the needle carrying hub with the needle extending axially from the carpule for administering an injection or release the needle carrying hub for retracting the cannula into its carpule to permit a safe disposal of the needle while avoiding an accidental and potentially life threatening needle strike.

US 3,144,178 A discloses a medical holder comprising: a body portion having a bore therethrough, a plunger having normally divergent arms slidable in said bore, the bore being of a diameter to snugly embrace the arms when they are parallel to one another and functioning to draw the arms together when they are drawn into said bore, said plunger having coupling means at the free ends of the arms for coupling with a piston located within one end of a cartridge and which cartridge at its other end has a hub portion, said body portion having spaced apart resilient legs extending therefrom, each leg terminating in a jaw directed toward the jaw on the other leg, each jaw having a bevelled outer face, said bevelled faces facilitating the positioning of the cartridge in the holder between the legs and engaging the hub portion of the cartridge when the resilient legs are brought together, and a sleeve slidable along the legs adapted when moved toward the jaws to cause the jaws to firmly engage the hub portion of the cartridge, the plunger coupling means automatically coupling with the piston.

WO 2011/001161 A1 discloses an apparatus for removing a sheath from a syringe. The sheath provides a sterile cover for a needle of the syringe. The apparatus comprises a substantially cylindrical housing defining an opening for receiving a sheath attached to a syringe, and a driver mounted on the housing and being slideable along the housing between first and second axially displaced positions. The apparatus further comprises a plurality of radially deflectable fingers mounted within said housing and being coupled to said driver for movement therewith. The fingers are configured such that movement of said driver from said first to said second position causes said fingers to slide over said sheath and engage with a formation on said sheath. Movement of the driver from said second position towards said first position causes said fingers to push the sheath off the syringe. Also provided is an injection device comprising the apparatus.

WO 2010/147553 A1 discloses a medicament container holder arrangement comprising a medicament container holder and a medicament container, wherein the medicament container holder is provided with distally arranged support surfaces for flanges arranged on a distal part of the medicament container to be placed in said medicament container holder. The medicament container is arranged with a needle shield to the proximal end thereof, wherein said needle shield has a diameter generally equal or larger than the diameter of said medicament container. The medicament container holder arrangement further comprises holders arranged on the container holder for holding said medicament container around a proximal shoulder portion thereof and a resilient support element arranged between the flanges and the support surfaces. Thereby the holders and the resilient support element absorb and spread forces between the medicament container and the container holder.

WO 2007/083115 A1 discloses an autoinjector comprising a housing, in which can be mounted a syringe comprising a barrel for holding a volume of medicament, a needle at one end of the barrel in fluid communication with the medicament and a plunger axially-moveable in the barrel to a forwardmost position. The autoinjector further comprises a syringe support means for supporting the barrel at an axial location at or forward of the forwardmost position of the plunger and having a reaction surface for the syringe. In use, said reaction surface provides an axial compressive force on said barrel when a forward axial force is applied to the plunger.

### Summary of the Invention

It is an object of the present invention to provide an improved syringe carrier.

The present invention provides a syringe carrier according to the appended independent claim 1 and a method for assembling a syringe according to the appended independent claim 13.

The syringe carrier according to the present invention comprises a body adapted to receive a barrel of a syringe. The body includes two sections having distal ends with shoulder sections adapted to engage a circumferential gap between the barrel of the syringe and a needle shield covering a needle of the syringe.

In an illustrative example, the sections are resiliently coupled to a collar on a proximal end of the body. The shoulder sections deflect when engaged by the needle shield and return to a non-deflected position when disengaged by the needle shield to engage the circumferential gap between the barrel of the syringe and the needle shield.

In the invention, the sections are resiliently coupled to a collar on a distal end of the body. In an exemplary embodiment, the sections deflect when engaged by the needle shield and return to a non-deflected position when disengaged by the needle shield to engage a finger flange of the syringe. The body includes resilient arms having additional shoulder sections adapted to engage the circumferential gap between the barrel of the syringe and a needle shield covering a needle of the syringe. The arms deflect when engaged by the needle shield and return to a non-deflected position when disengaged by the needle shield to engage the circumferential gap between the barrel of the syringe and a needle shield.

In an illustrative example, the sections are coupled via at least one hinge and are movable between an open position and a closed position. A first section includes a pin adapted to engage a hole on a second section to secure the sections in the closed position.

In an illustrative example, the sections are coupled via at least one clip and are movable between an open position and a closed position. The at least one clip includes a hook on a first section adapted to engage an eye on a second section to secure the sections in the closed position.

In an illustrative example, the sections include doors hingedly coupled to the body and additional shoulder sections are formed on distal ends of the doors.

In an exemplary embodiment, the shoulder sections include proximally-facing contoured surfaces to accommodate a proximal portion of a neck of the syringe and distally-facing planar surfaces to abut the needle shield.

In an exemplary embodiment, the body includes one or more viewing windows.

In an exemplary embodiment, the body includes a retainer element adapted to provide an abutment surface to prevent the syringe from disengaging the syringe carrier in a proximal direction.

Further scope of applicability of the present invention will become apparent from the detailed description given hereinafter. However, it should be understood that the detailed description and specific examples, while indicating preferred embodiments of the invention, are given by way of illustration only, since various changes and modifications within the scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a top view of an illustrative example of a syringe carrier,
- Figure 2: is a lateral view of the syringe carrier of figure 1,
- Figure 3: is a longitudinal section of the syringe carrier of figure 1 in the section plane A-A,
- Figure 4: is a perspective view of the syringe carrier of figure 1,
- Figure 5: is a top view of an exemplary embodiment of a syringe carrier according to the present invention,
- Figure 6: is a lateral view of the syringe carrier of figure 5,
- Figure 7: is a longitudinal section of the syringe carrier of figure 5 in the section plane A-A,
- Figure 8: is a perspective view of the syringe carrier of figure 5,
- Figure 9: is a top view of another illustrative example of a syringe carrier,
- Figure 10: is a lateral view of the syringe carrier of figure 9,
- Figure 11: is a longitudinal section of the syringe carrier of figure 9 in the section plane A-A,
- Figure 12: is a perspective view of the syringe carrier of figure 9,
- Figure 13: is another perspective view of the syringe carrier of figure 9 with a syringe inserted,
- Figure 14: is a top view of yet another illustrative example of a syringe carrier,
- Figure 15: is a lateral view of the syringe carrier of figure 14,
- Figure 16: is a longitudinal section of the syringe carrier of figure 14 in the section plane A-A,
- Figure 17: is a perspective view of the syringe carrier of figure 14,
- Figure 18: is another perspective view of the syringe carrier of figure 14 with a syringe inserted,
- Figure 19: is a top view of yet another illustrative example of a syringe carrier,
- Figure 20: is a lateral view of the syringe carrier of figure 19,
- Figure 21: is a longitudinal section of the syringe carrier of figure 19 in the section plane A-A,
- Figure 22: is a perspective view of the syringe carrier of figure 19,
- Figure 23: is another perspective view of the syringe carrier of figure 19 with a syringe inserted,
- Figure 24: is a top view of yet another illustrative example of a syringe carrier,
- Figure 25: is a lateral view of the syringe carrier of figure 24,
- Figure 26: is a longitudinal section of the syringe carrier of figure 24 in the section plane A-A,
- Figure 27: is a perspective view of the syringe carrier of figure 24,
- Figure 28: is another perspective view of the syringe carrier of figure 24 with a syringe inserted,
- Figure 29: is a top view of yet another illustrative example of a syringe carrier,
- Figure 30: is a lateral view of the syringe carrier of figure 29,
- Figure 31: is a longitudinal section of the syringe carrier of figure 29 in the section plane A-A,
- Figure 32: is a perspective view of the syringe carrier of figure 29,
- Figure 33: is another perspective view of the syringe carrier of figure 29 with a syringe inserted,
- Figure 34: is a top view of yet another illustrative example of a syringe carrier,
- Figure 35: is a lateral view of the syringe carrier of figure 34,
- Figure 36: is a longitudinal section of the syringe carrier of figure 34 in the section plane A-A,
- Figure 37: is a perspective view of the syringe carrier of figure 34,
- Figure 38: is another perspective view of the syringe carrier of figure 34 with a syringe inserted,
- Figure 39: is a top view of yet another illustrative example of a syringe carrier,
- Figure 40: is a lateral view of the syringe carrier of figure 39,
- Figure 41: is a longitudinal section of the syringe carrier of figure 39 in the section plane B-B,
- Figure 42: is a perspective view of the syringe carrier of figure 39,
- Figure 43: is another perspective view of the syringe carrier of figure 39 with a syringe inserted,
- Figure 44: is a top view of yet another illustrative example of a syringe carrier,
- Figure 45: is a lateral view of the syringe carrier of figure 44,
- Figure 46: is a longitudinal section of the syringe carrier of figure 44 in the section plane B-B,
- Figure 47: is a perspective view of the syringe carrier of figure 44, and
- Figure 48: is another perspective view of the syringe carrier of figure 44 with a syringe inserted.

Corresponding parts are marked with the same reference symbols in all figures.

### Detailed Description

Generally, and applicable to all examples and embodiments of the present invention, the syringe 2 comprises a barrel 2.1 and a neck 2.2 which has a smaller diameter than the barrel 2.1. A needle 3 is mounted to the neck 2.2 and a rigid needle shield (RNS) 4 is removably arranged on the needle 3. When coupled to the needle 3, a portion of the RNS may cover a portion of the neck 2.2, leaving a circumferential gap between the barrel 2.1 and the RNS 4. The RNS 4 has a diameter substantially equal to the diameter of the barrel 2.1.

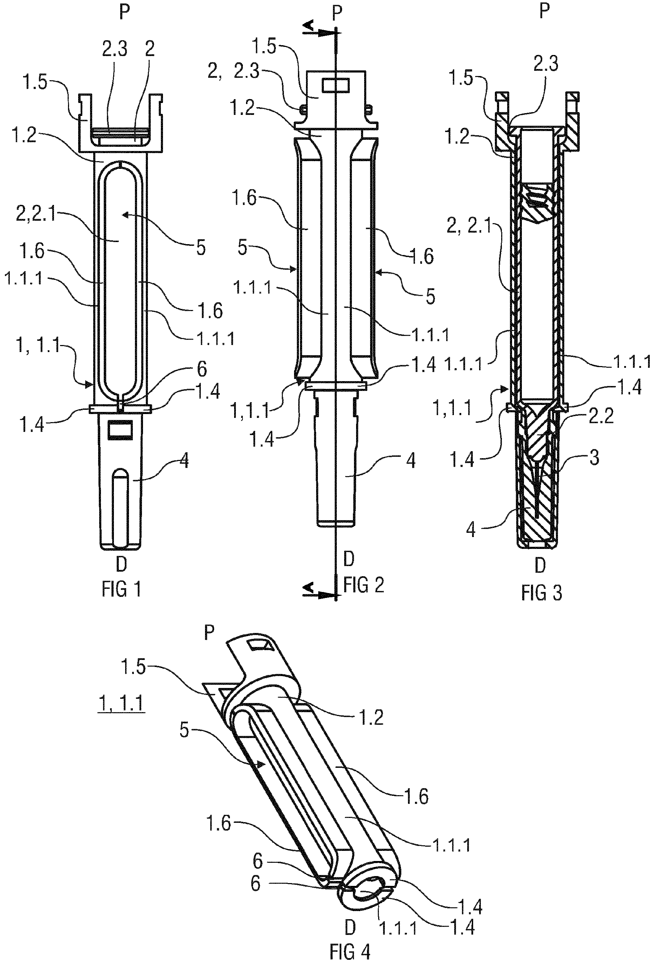

Figures 1-4 show an illustrative example of a syringe carrier 1. Figure 1 is a top view of the syringe carrier 1 for supporting a syringe 2. Figure 2 is a lateral view of the syringe carrier of figure 1. Figure 3 is a longitudinal section of the syringe carrier of figure 1 in the section plane A-A. Figure 4 is a perspective view of the syringe carrier of figure 1 without the syringe 2.

As shown in Figures 1-4, the syringe carrier 1 comprises an elongate body 1.1 arranged to receive the barrel 2.1. In this example, the body 1.1 has a cylindrical shape with an internal diameter corresponding to the diameter of the barrel 2.1. The body 1.1 comprises a collar 1.2 at a proximal end dimensioned to allow axial insertion of the syringe 2 into the syringe carrier 1 in a distal direction D. Resilient sections 1.1.1 extend distally from the collar 1.2. Distal ends of the sections 1.1.1 include shoulder sections 1.4 shaped as portions of a circle arranged in a transverse plane with respect to a longitudinal axis of the carrier 1. The shoulder sections include facing surfaces 6. When the sections 1.1.1 are in a non-deflected position, the facing surfaces 6 may abut each other, and the shoulder sections 1.4 form a circular shoulder (because the facing surfaces 6 abut each other) adapted to engage the circumferential gap between the barrel 2.1 and the RNS 4.

The syringe 2, with RNS 4 attached to the needle 3, may be loaded into the syringe carrier 1 by sliding the syringe 2 in the distal direction D into the syringe carrier 1. When the RNS 4 abuts the shoulder sections 1.4, additional axial force may be applied to cause the arms 1.3 to deflect radially. When the RNS 4 has bypassed the shoulder sections 1.4, the sections 1.1.1 may return to the non-deflected position, and the shoulder sections 1.4 may engage the circumferential gap between the barrel 2.1 and the RNS 4 and prevent the syringe 2 from moving in the distal direction D relative to the syringe carrier 1.

In an embodiment, the proximal end 1.5 of the body 1.1 may be arranged to receive a finger flange 2.3 of the syringe 2.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the arms 1.3 are substantially contiguous when the arms 1.3 are in the non-deflected position (as shown in Figure 1). A projection 1.6 may be formed around each cut-out, and when the sections 1.1.1 are in the non-deflected position, the projections 1.6 may form an outline for the window 5. In another exemplary embodiment, the windows 5 may be formed in the sections 1.1.1.

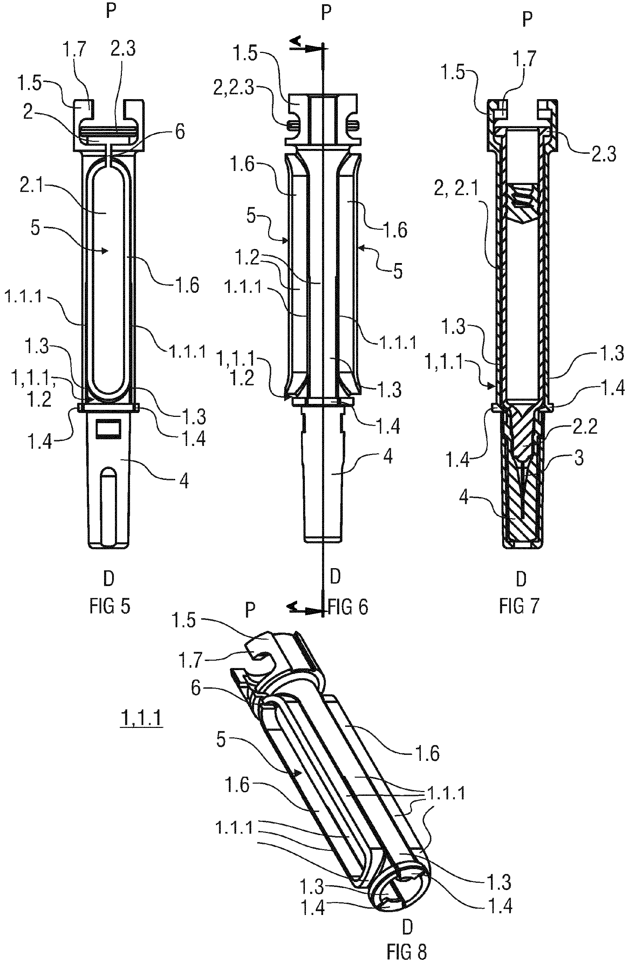

Figures 5-8 show an exemplary embodiment of a syringe carrier 1 according to the present invention. Figure 6 is a lateral view of the syringe carrier 1 of figure 5. Figure 7 is a longitudinal section of the syringe carrier 1 of figure 5 in the section plane A-A. Figure 8 is a perspective view of the syringe carrier of figure 5 without the syringe 2.

As shown in Figures 5-8, the syringe carrier 1 comprises an elongate body 1.1 arranged to receive the barrel 2.1. In this exemplary embodiment, the body 1.1 is comprised of two resilient sections 1.1.1 which, when together, have a cylindrical shape with an internal diameter corresponding to the diameter of the barrel 2.1. Distal ends of the sections 1.1.1 of the body 1.1 comprise part of a collar 1.2 dimensioned to allow axial insertion of the syringe 2 into the syringe carrier 1. Resilient arms 1.3 are formed in the body 1.1. Distal ends of the arms 1.3 include shoulder sections 1.4 shaped as portions of a circle arranged in a transverse plane with respect to a longitudinal axis of the carrier 1. The shoulder sections include facing surfaces 6. When the arms 1.3 are in a non-deflected position, the facing surfaces 6 may abut the distal ends of the sections 1.1.1 of the body 1.1 to form a circular shoulder adapted to engage the circumferential gap between the barrel 2.1 and the RNS 4.

The syringe 2, with RNS 4 attached to the needle 3, may be loaded into the syringe carrier 1 by sliding the syringe 2 in the distal direction D into the syringe carrier 2. When the RNS 4 abuts proximal ends of the sections 1.1.1, the sections 1.1.1 may deflect radially. When the RNS 4 has bypassed the proximal ends of the section 1.1.1, the sections 1.1.1 may return to the non-deflected position. When the RNS 4 abuts the shoulder sections 1.4, the arms 1.3 may deflect until the RNS 4 bypasses the shoulder sections 1.4. Then, the arms 1.3 may return to the non-deflected position, and the shoulder sections 1.4 and the collar 1.2 may engage the circumferential gap between the barrel 2.1 and the RNS 4 and prevent the syringe 2 from moving in the distal direction D relative to the syringe carrier 1.

In an exemplary embodiment, the proximal end 1.5 of the body 1.1 may be arranged to receive a finger flange 2.3 of the syringe 2. The proximal end 1.5 may also include a retainer element 1.7 which is adapted to provide an abutment surface to prevent the syringe 2 from disengaging the syringe carrier 1 in the proximal direction D.

In an exemplary embodiment, the shoulder sections 1.4 may include proximally-facing contoured surfaces to accommodate a proximal portion of the neck 2.2 of the syringe 2 and distally-facing planar surfaces to abut the RNS 4.

In an exemplary embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the sections 1.1.1 are substantially contiguous when the sections 1.1.1 are in the non-deflected position (as shown in Figure 5). A projection 1.6 may be formed around each cut-out, and when the sections 1.1.1 are in the non-deflected position, the projections 1.6 may form an outline for the window 5.

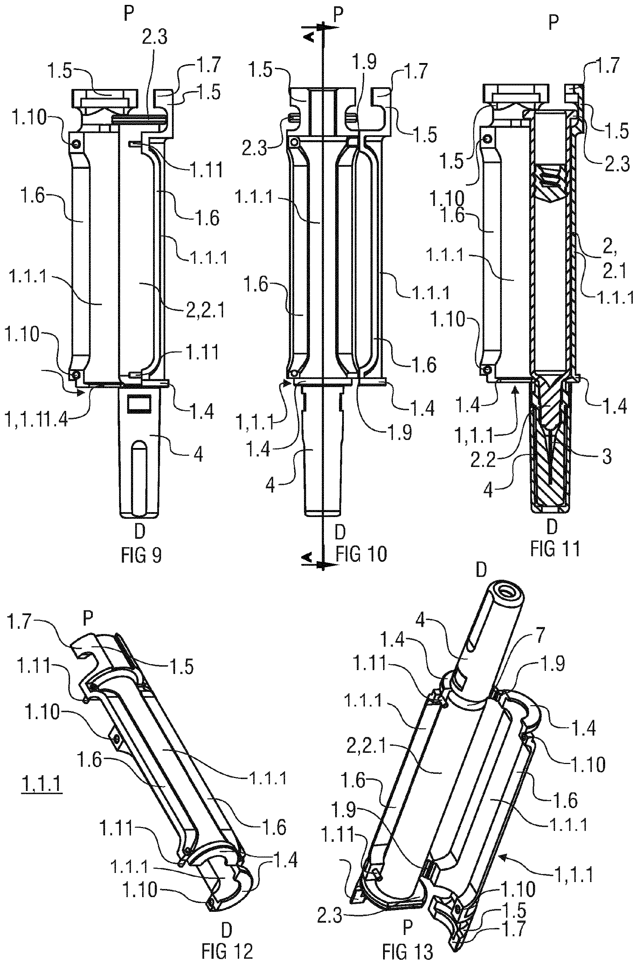

Figures 9-13 show another illustrative example of a syringe carrier 1. Figure 9 is a top view of an example of a syringe carrier 1 for supporting a syringe 2. Figure 10 is a lateral view of the syringe carrier 1 of figure 9. Figure 11 is a longitudinal section of the syringe carrier 1 of figure 9 in the section plane A-A. Figure 12 is a perspective view of the syringe carrier of figure 9 without the syringe 2. Figure 13 is another perspective view of the syringe carrier of figure 9.

As shown in Figures 9-13, the syringe carrier 1 comprises an elongate body 1.1 arranged to receive the barrel 2.1. In this example, the body 1.1 is comprised of two sections 1.1.1 which, when together, have a cylindrical shape with an internal diameter corresponding to the diameter of the barrel 2.1. The sections 1.1.1 may be coupled by a side hinge which allows the section 1.1.1 to rotate relative to each other sufficient to receive the syringe 2. Proximal and distal ends of the sections 1.1.1 include shoulder sections 1.4 shaped as portions of a circle arranged in a transverse plane with respect to a longitudinal axis of the carrier 1. The shoulder sections include facing surfaces 6.

When the sections 1.1.1 are in a closed position, the facing surfaces 6 may abut each other so that the shoulder sections 1.4 form circular shoulders adapted to proximally abut a finger flange 2.3 on the syringe 2 and to distally engage the circumferential gap between the barrel 2.1 and the RNS 4. The facing surfaces 6 of one section 1.1.1 may include holes 1.10 and the facing surfaces 6 of the other section 1.1.1 may include pins 1.11 adapted to engage (e.g., frictionally, snap-fit, etc.) the holes 1.10 to secure the sections 1.1.1 in the closed position.

The syringe 2, with RNS 4 attached to the needle 3, may be loaded into the syringe carrier 1 by opening the sections 1.1.1 about the hinge and placing the syringe 2 in the syringe carrier 2. When the sections 1.1.1 are closed, the pins 1.11 engage the holes 1.10, and the proximal shoulder sections 1.4 form circular shoulders adapted to proximally abut a finger flange 2.3 on the syringe 2 and the distal shoulder sections 1.4 to distally engage the circumferential gap between the barrel 2.1 and the RNS 4. Thus, the syringe 2 is prevented from moving axially relative to the syringe carrier 1.

In an embodiment, the proximal end 1.5 may include a retainer element 1.7 which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, viewing windows 5 may be arranged in the body 1.1 for allowing visual access to the barrel 2.1 of the syringe 2 when the syringe 2 is in the syringe carrier 2. In an exemplary embodiment, the windows 5 are formed when cut-outs in the sections 1.1.1 are substantially contiguous when the sections 1.1.1 are in the closed position. A projection 1.6 may be formed around each cut-out, and when the sections 1.1.1 are in the non-deflected position, the projections 1.6 may form an outline for the window 5.

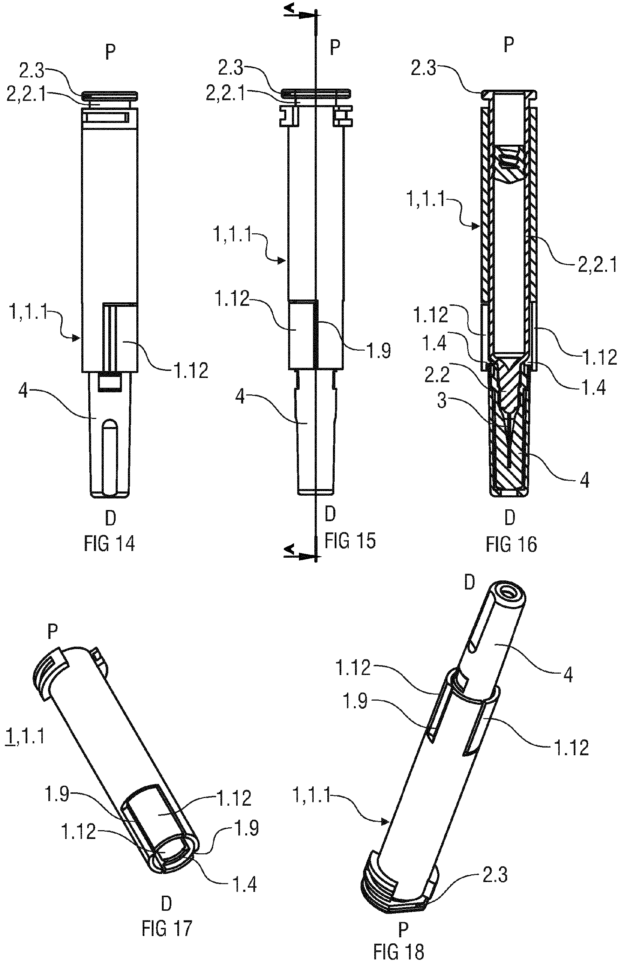

Figures 14-18 show another illustrative example of a syringe carrier 1. Figure 14 is a top view of an example of a syringe carrier 1 for supporting a syringe 2. Figure 15 is a lateral view of the syringe carrier 1 of figure 14. Figure 16 is a longitudinal section of the syringe carrier 1 of figure 14 in the section plane A-A. Figure 17 is a perspective view of the syringe carrier of figure 14 without the syringe 2. Figure 18 is another perspective view of the syringe carrier of figure 14.

As shown in Figures 14-18, the syringe carrier 1 comprises an elongate body 1.1 arranged to receive the barrel 2.1. In this example, the body 1.1 has a cylindrical shape with an internal diameter corresponding to the diameter of the barrel 2.1. A distal end of the body 1.1 includes a shoulder section 1.4 shaped as a portion of a circle arranged in a transverse plane with respect to a longitudinal axis of the carrier 1, and at least one door 1.12 hingedly coupled to the body 1.1 and including a shoulder section 1.4. A hinge 1.9 coupling the door 1.12 to the body 1.1 may be provided on an axis parallel to the longitudinal axis of the syringe carrier 1 or on an axis transverse to the longitudinal axis of the syringe carrier 1. The shoulder section 1.4 includes facing surfaces 6 which abut facing surfaces 6 of the door 1.12 when the door 1.12 is in a closed position (as shown in Figure 14). When the door 1.12 is in the closed position, the facing surfaces 6 may abut each other so that the shoulder sections 1.4 on the body 1.1 and the door 1.12 to form a circular shoulder adapted to engage the circumferential gap between the barrel 2.1 and the RNS 4. The facing surfaces 6 of the door 1.12 may include holes 1.10 and the facing surfaces 6 of the body 1.1 may include pins 1.11 (or vice-versa) adapted to engage (e.g., frictionally, snap-fit, etc.) the holes 1.10 to secure the door 1.12 in the closed position.

The syringe 2, with RNS 4 attached to the needle 3, may be loaded into the syringe carrier 1 by opening the door 1.12 and sliding the syringe 2 into the syringe carrier 1. When the circumferential gap between the barrel 2.1 and the RNS 4 engages the shoulder section 1.4 on the body 1.1, the door 1.12 may be closed to engage the gap and prevent the syringe 2 from moving axially relative to the syringe carrier 1.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, viewing windows (not shown) may be arranged in the body 1.1 for allowing visual access to the barrel 2.1 of the syringe 2 when the syringe 2 is in the syringe carrier 2. In an exemplary embodiment, the windows are formed as cut-outs.

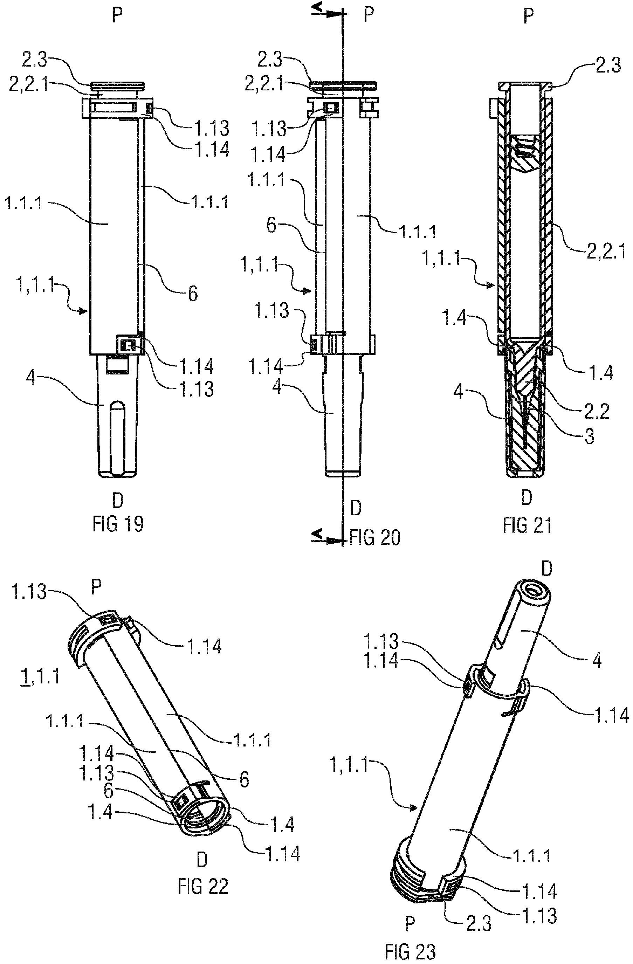

Figures 19-23 show another illustrative example of a syringe carrier 1. Figure 19 is a top view of an example of a syringe carrier 1 for supporting a syringe 2. Figure 20 is a lateral view of the syringe carrier 1 of figure 19. Figure 21 is a longitudinal section of the syringe carrier 1 of figure 19 in the section plane A-A. Figure 22 is a perspective view of the syringe carrier of figure 19 without the syringe 2. Figure 23 is another perspective view of the syringe carrier of figure 19.

As shown in Figures 19-23, the syringe carrier 1 comprises an elongate body 1.1 arranged to receive the barrel 2.1. In this example, the body 1.1 is comprised of two sections 1.1.1 which, when together, have a cylindrical shape with an internal diameter corresponding to the diameter of the barrel 2.1. The sections 1.1.1 may be coupled together by clips. In an exemplary embodiment, a clip may comprise an eye 1.14 on a first section adapted to engage a hook 1.13 on a second section. The eye 1.14 may have a cross-section substantially equal to the cross-section of the hook 1.13 such that the eye 1.14 and hook 1.13 engage in a snap-fit. Distal ends of the sections 1.1.1 include shoulder sections 1.4 shaped as portions of a circle arranged in a transverse plane with respect to a longitudinal axis of the carrier 1. The shoulder sections include facing surfaces 6. When the sections 1.1.1 are in a closed position, the facing surfaces 6 may abut each other so that the shoulder sections 1.4 form circular shoulders adapted engage the circumferential gap between the barrel 2.1 and the RNS 4. Those of skill in the art will understand that the sections 1.1.1 may be hingedly connected.

The syringe 2, with RNS 4 attached to the needle 3, may be loaded into the syringe carrier 1 by opening the sections 1.1.1 and placing the syringe 2 in the syringe carrier 2. When the sections 1.1.1 are closed, the eyes 1.14 engage the hooks 1.13 and the shoulder sections 1.4 engage the circumferential gap between the barrel 2.1 and the RNS 4. Thus, the syringe 2 is prevented from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, viewing windows may be arranged in the body 1.1 for allowing visual access to the barrel 2.1 of the syringe 2 when the syringe 2 is in the syringe carrier 2.

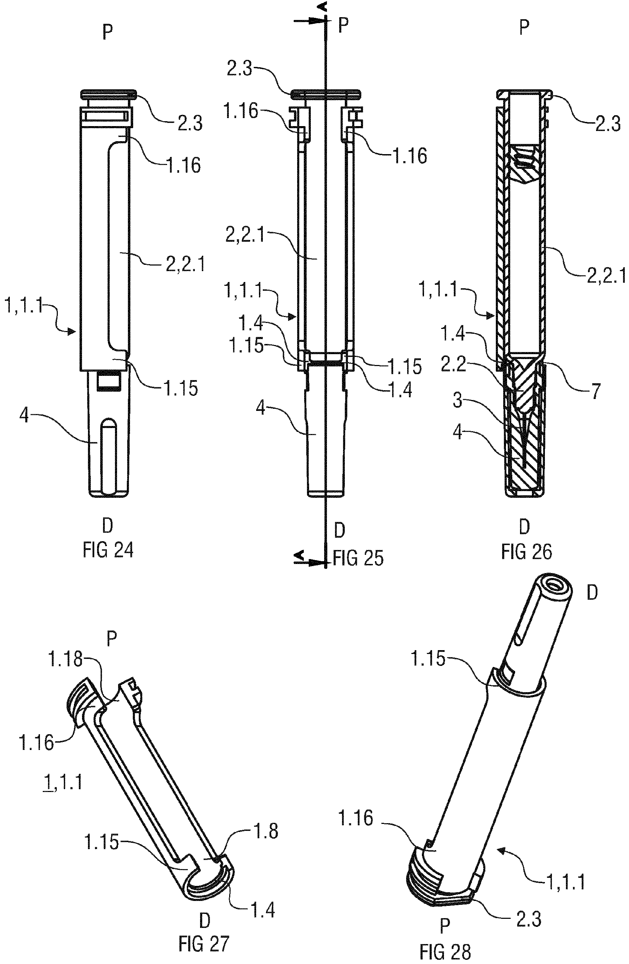

Figures 24-28 show another illustrative example of a syringe carrier 1. Figure 24 is a top view of an example of a syringe carrier 1 for supporting a syringe 2. Figure 25 is a lateral view of the syringe carrier 1 of figure 24. Figure 26 is a longitudinal section of the syringe carrier 1 of figure 24 in the section plane A-A. Figure 27 is a perspective view of the syringe carrier of figure 24 without the syringe 2. Figure 28 is another perspective view of the syringe carrier of figure 24.

As shown in Figures 24-28, the syringe carrier 1 comprises an elongate body 1.1 arranged to receive the barrel 2.1. In this example, the body 1.1 has a partially cylindrical shape with an internal diameter corresponding to the diameter of the barrel 2.1. The body 1.1 may include a longitudinal slot (e.g., a cut-out) which is adapted to snap over the barrel 2.1 of the syringe 2. Proximal and distal ends of the body 1.1 include clamps 1.15, 1.16 which are adapted to retain the syringe 2 when in the syringe carrier 1. The distal end of the body 1 further includes shoulder sections 1.4 shaped as a portion of a circle arranged in a transverse plane with respect to a longitudinal axis of the carrier 1. The shoulder sections 1.4 form circular shoulders adapted to engage the circumferential gap between the barrel 2.1 and the RNS 4.

The syringe 2, with RNS 4 attached to the needle 3, may be loaded into the syringe carrier 1 by pressing the barrel 2.1 against the clamps 1.15, 1.16, causing the clamps 1.15, 1.16 to deflect and widen the longitudinal slot in the body 1.1. When the barrel 2.1 bypasses the clamps 1.15, 1.16, the clamps 1.15, 1.16 return to their non-deflected position and retain the syringe 2 in the syringe carrier 1. The shoulder sections 1.4 engage the circumferential gap between the barrel 2.1 and the RNS 4. Thus, the syringe 2 is prevented from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, a viewing window may be arranged in the body 1.1 for allowing visual access to the barrel 2.1 of the syringe 2 when the syringe 2 is in the syringe carrier 2.

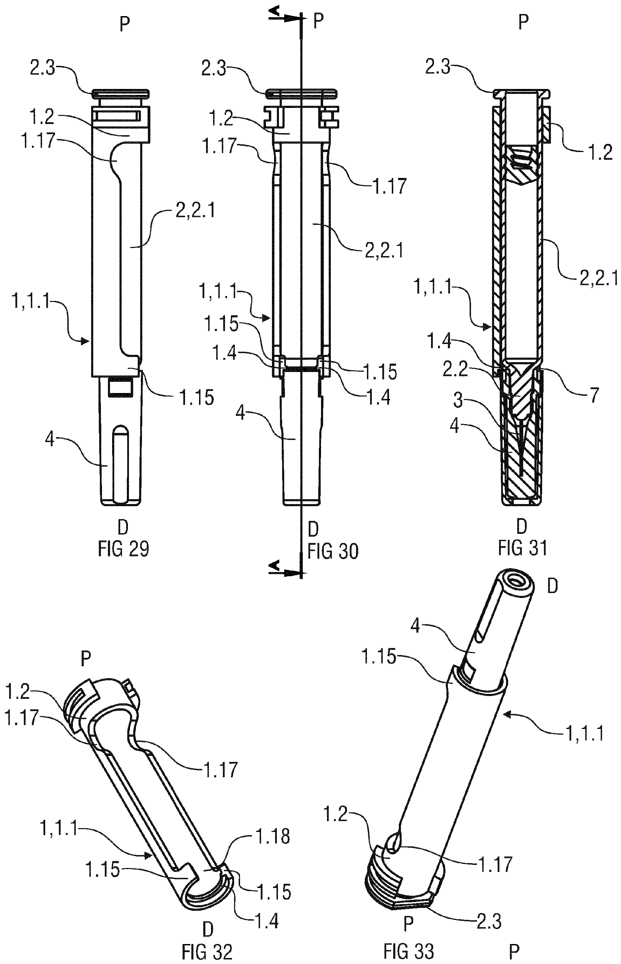

Figures 29-33 show another illustrative example of a syringe carrier 1. Figure 29 is a top view of an example of a syringe carrier 1 for supporting a syringe 2. Figure 30 is a lateral view of the syringe carrier 1 of figure 29. Figure 31 is a longitudinal section of the syringe carrier 1 of figure 29 in the section plane A-A. Figure 32 is a perspective view of the syringe carrier of figure 29 without the syringe 2. Figure 33 is another perspective view of the syringe carrier of figure 29.

As shown in Figures 29-33, the syringe carrier 1 comprises an elongate body 1.1 arranged to receive the barrel 2.1. In this example, the body 1.1 has a partially cylindrical shape with an internal diameter corresponding to the diameter of the barrel 2.1. The body 1.1 includes a collar 1.2 at its proximal end and may include a longitudinal slot (e.g., a cut-out) formed in the body 1.1 distally of the collar 1.2 which is adapted to snap over the barrel 2.1 of the syringe 2. A pair of groove hinges 1.17 may be formed in the body 1.1 adjacent a proximal end of the slot. The distal end of the body 1 includes shoulder sections 1.4 shaped as a portion of a circle arranged in a transverse plane with respect to a longitudinal axis of the carrier 1. The shoulder sections 1.4 form circular shoulders adapted to engage the circumferential gap between the barrel 2.1 and the RNS 4.

The syringe 2, with RNS 4 attached to the needle 3, may be loaded into the syringe carrier 1 by sliding the syringe 2 through the collar 1.2 in the distal direction D. When the RNS 4 abuts the shoulder sections 1.4, the body 1.1 may radially deflect (e.g., rotate) about the groove hinges 1.17. When the RNS 4 bypasses the shoulder sections 1.4, the body 1.1 may return to its non-deflected position and retain the syringe 2 in the syringe carrier 1. The shoulder sections 1.4 engage the circumferential gap between the barrel 2.1 and the RNS 4. Thus, the syringe 2 is prevented from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, a viewing window may be arranged in the body 1.1 for allowing visual access to the barrel 2.1 of the syringe 2 when the syringe 2 is in the syringe carrier 2.

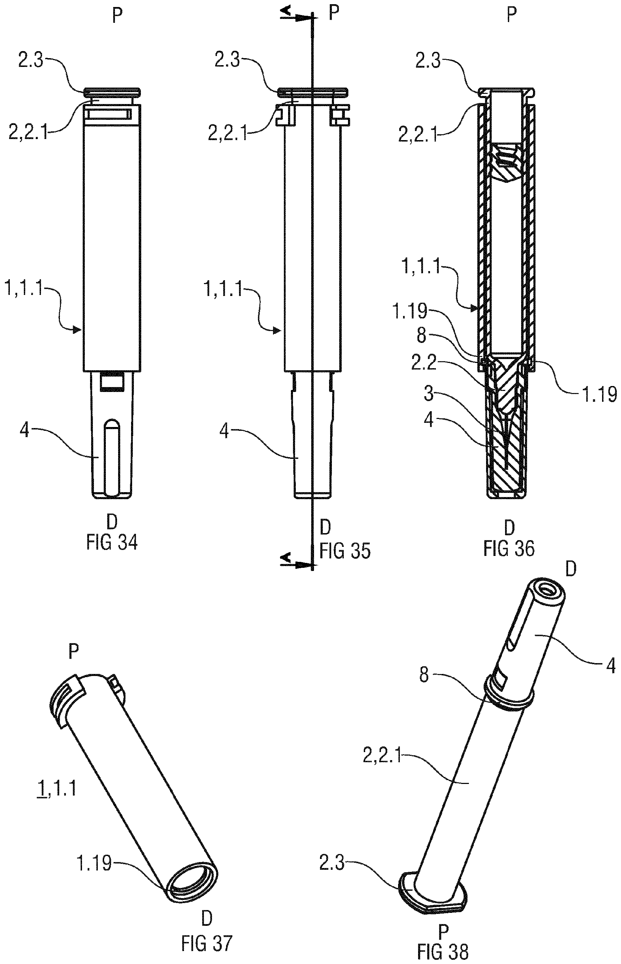

Figures 34-38 show another illustrative example of a syringe carrier 1. Figure 34 is a top view of an example of a syringe carrier 1 for supporting a syringe 2. Figure 35 is a lateral view of the syringe carrier 1 of figure 34. Figure 36 is a longitudinal section of the syringe carrier 1 of figure 34 in the section plane A-A. Figure 37 is a perspective view of the syringe carrier of figure 34 without the syringe 2. Figure 38 is another perspective view of the syringe carrier of figure 34.

As shown in Figures 34-38, the syringe carrier 1 comprises an elongate body 1.1 arranged to receive the barrel 2.1. In this example, the body 1.1 has a cylindrical shape with an annular groove 1.19 adjacent its distal end which is adapted to engage a circlip 8. The circlip 8 may engage the circumferential gap between the barrel 1.2 and the RNS 4.

The syringe 2, with RNS 4 attached to the needle 3 and the circlip 8 attached to the syringe 2, may be loaded into the syringe carrier 1 by sliding the syringe 2 into the syringe carrier 1 in the distal direction D. In a non-deflected position, an outer diameter of the circlip 8 may be substantially equal to a diameter of the body 1.1. Thus, when the syringe 2 with the circlip 8 is inserted into the syringe carrier 1, the circlip 8 may deflect radially until the circlip 8 reaches the annular groove 1.19. The circlip 8 may then expand to the non-deflected position and retain the syringe 2 in an axial position relative to the syringe carrier 1. That is, the circlip 8 may engage the annular groove 1.19 and the circumferential gap between the barrel 2.1 and the RNS 4. Thus, the syringe 2 is prevented from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, a viewing window may be arranged in the body 1.1 for allowing visual access to the barrel 2.1 of the syringe 2 when the syringe 2 is in the syringe carrier 2.

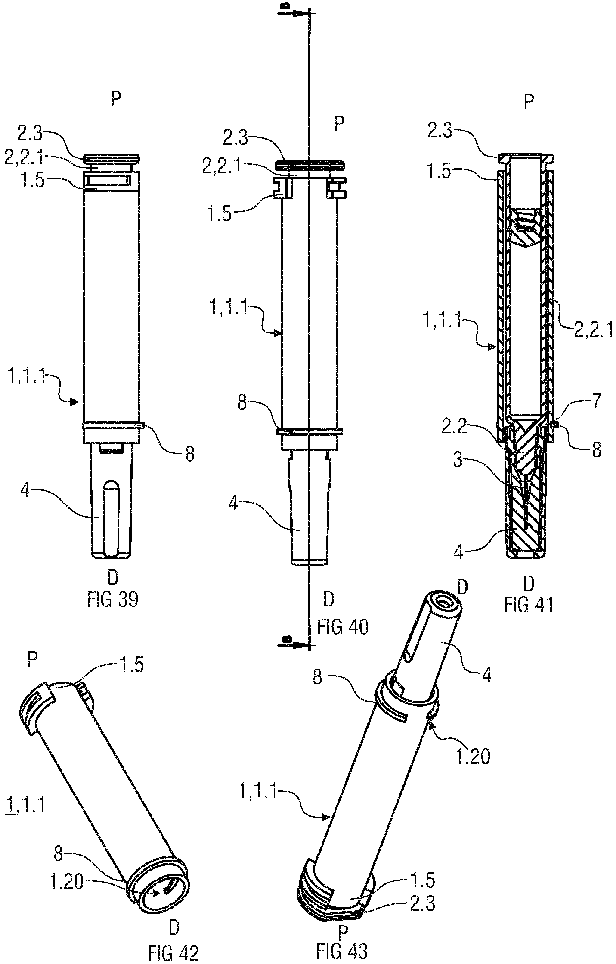

Figures 39-43 show another illustrative example of a syringe carrier 1. Figure 39 is a top view of an example of a syringe carrier 1 for supporting a syringe 2. Figure 40 is a lateral view of the syringe carrier 1 of figure 39. Figure 41 is a longitudinal section of the syringe carrier 1 of figure 39 in the section plane A-A. Figure 42 is a perspective view of the syringe carrier of figure 39 without the syringe 2. Figure 43 is another perspective view of the syringe carrier of figure 39.

As shown in Figures 39-43, the syringe carrier 1 comprises an elongate body 1.1 arranged to receive the barrel 2.1. In this example, the body 1.1 has a cylindrical shape with an annular groove 1.19 having at least one aperture 1.20 adjacent its distal end which is adapted to engage a circlip 8.

The syringe 2, with RNS 4 attached to the needle 3, may be loaded into the syringe carrier 1 by sliding the syringe 2 into the syringe carrier 1 in the distal direction D. When the circumferential gap between the barrel 2.1 and the RNS 4 is aligned with the annular groove 1.19, the circlip 8 may be coupled to the body 1.1 and engage the apertures 1.20. By extending inwardly through the apertures, the circlip 8 may be coupled to the outside of the body 1.1 but engage the circumferential gap between the barrel 2.1 and the RNS 4. The engagement between the circlip 8 and the apertures 1.20 prevents the circlip 8 from translating relative to the body 1.1, and the engagement between the circlip 8 and the circumferential gap prevents the syringe 2 from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the shoulder sections 1.4 may include proximally-facing contoured surfaces to accommodate a proximal portion of the neck 2.2 of the syringe 2 and distally-facing planar surfaces to abut the RNS 4.

In an embodiment, a viewing window may be arranged in the body 1.1 for allowing visual access to the barrel 2.1 of the syringe 2 when the syringe 2 is in the syringe carrier 2.

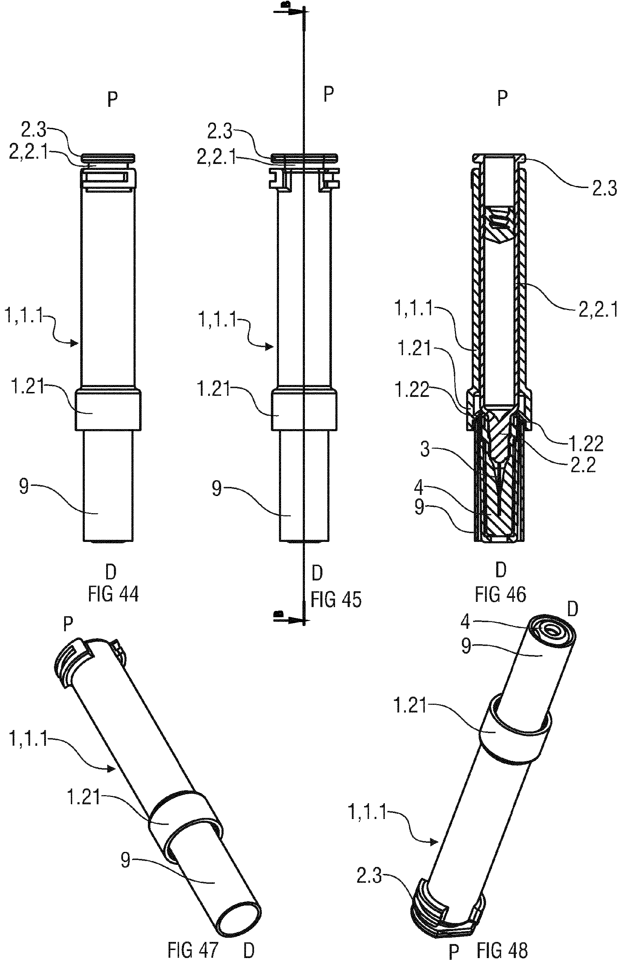

Figures 44-48 show another illustrative example of a syringe carrier 1 and a tool 9 for inserting a syringe 2 into the syringe carrier 1.

As shown in Figures 39-43, the syringe carrier 1 comprises an elongate body 1.1 arranged to receive the barrel 2.1. In this example, the body 1.1 has an enlarged portion 1.21 on its distal end. The body 1.1 has cylindrical shape with a first diameter and the enlarged portion 1.21 has a second diameter, larger than the first diameter. The enlarged portion 1.21 has one or more resilient barbs 1.22 extending toward a longitudinal axis of the body 1.1 and angled toward a proximal end of the body 1.1.

The syringe 2, with RNS 4 attached to the needle 3, may be loaded into the syringe carrier 1 by inserting the tool 9 into the enlarged portion 1.21 of the syringe carrier 1. The tool 9 may be a cylinder having an open end adapted to receive the RNS 4. The tool 9 may have a third diameter substantially equal to the second diameter. As the tool 9 is inserted into the enlarged portion 1.21, the tool 9 engages and deflects the resilient barbs 1.22. When the barbs 1.22 are deflected, the RNS 4 can pass the barbs 1.22 in the distal direction D and extend from a distal opening of the body 1.1. When a finger flange 2.3 of the syringe 2 abuts a proximal end of the body 1.1, the tool 9 may be removed and the barbs 1.22 may engage the circumferential gap between the barrel 2.1 and the RNS 4 to prevent the syringe 2 from moving axially relative to the syringe carrier 1.

In an embodiment, the proximal end may include a retainer element which is adapted to provide an abutment surface to prevent the syringe 2 from disengaging the syringe carrier 1 in the proximal direction D.

In an embodiment, the barbs 1.22 may include proximally-facing contoured surfaces to accommodate a proximal portion of the neck 2.2 of the syringe 2 and distally-facing planar surfaces to abut the RNS 4.

In an embodiment, a viewing window may be arranged in the body 1.1 for allowing visual access to the barrel 2.1 of the syringe 2 when the syringe 2 is in the syringe carrier 2.

It is apparent to those skilled in the art that the number of deflectable arms 1.3, shoulder sections 1.4, clips 8 may be varied without departing from the scope of the invention. Likewise, all the illustrated embodiments may be implemented with or without viewing windows 5, projections 1.6, restraining features retainer elements 1.7 and clips. Different kinds of clips may likewise be applied.

## Claims

1. A syringe carrier (1) comprising:
a syringe (2) having a barrel (2.1) and a needle shield (4) covering a needle (3) arranged on a distal end of the syringe (2), wherein a circumferential gap is provided between the barrel (2.1) of the syringe (2) and the needle shield (4), and
a body (1.1), wherein the barrel (2.1) is received in the body (1.1), the body (1.1) including at least two sections (1.1.1) having distal ends with shoulder sections (1.4) shaped as portions of a circle arranged in a transverse plane with respect to a longitudinal axis of the syringe carrier (1), wherein the sections (1.1.1) are resiliently coupled to a collar (1.2) on a distal end of the body (1.1), and wherein the shoulder sections (1.4) are adapted to engage the circumferential gap to prevent the syringe (2) from moving in a distal direction (D) relative to the syringe carrier (1).

2. The syringe carrier (1) according to claim 1, wherein the sections (1.1.1) deflect when engaged by the needle shield (4) and return to a non-deflected position when disengaged by the needle shield (4) to engage a finger flange (2.3) of the syringe (2).

3. The syringe carrier (1) according to any one of the preceding claims, wherein the shoulder sections (1.4) include proximally-facing contoured surfaces to accommodate a proximal portion of a neck (2.2) of the syringe (2) and distally-facing planar surfaces to abut the needle shield (4).

4. The syringe carrier (1) according to any one of the preceding claims, wherein the body (1.1) includes one or more viewing windows (5).

5. The syringe carrier (1) according to claim 4, wherein the viewing windows (5) are formed when cut-outs in the sections (1.1.1) are substantially contiguous when the sections (1.1.1) are in the non-deflected position.

6. The syringe carrier (1) according to claim 4, wherein a projection (1.6) is formed around each cut-out, and when the sections (1.1.1) are in the non-deflected position, the projections (1.6) form an outline for the viewing window (5).

7. The syringe carrier (1) according to any one of the preceding claims, wherein the body (1.1) includes a retainer element (1.7) adapted to provide an abutment surface to prevent the syringe (2) from disengaging the syringe carrier (1) in a proximal direction (P).

8. The syringe carrier (1) according to any one of the preceding claims, wherein the two resilient sections (1.1.1), when together, have a cylindrical shape with an internal diameter corresponding to the diameter of the barrel (2.1).

9. The syringe carrier (1) according to any one of the preceding claims, wherein the collar (1.2) is partly formed by distal ends of the sections (1.1.1) of the body (1.1), the collar (1.2) being dimensioned to allow axial insertion of the syringe (2) into the syringe carrier (1).

10. Medicament delivery device comprising the syringe carrier (1) according to any one of the preceding claims.

11. Medicament delivery device according to claim 10, wherein the needle is the needle (3) of the syringe (2), and wherein the shoulder sections (1.4) engage the circumferential gap.

12. Medicament delivery device according to claim 11, wherein the syringe (2) is pre-filled with a medicament.

13. Method for assembling a syringe (2) having a barrel (2.1) and a needle shield (4) covering a needle (3) of the syringe (2) into a syringe carrier (1) according to any one of claims 1 to 9, **characterized in that** the method comprises: sliding the syringe (2) in the distal direction (D) into the syringe carrier (1), wherein, when the needle shield (4) abuts proximal ends of the sections (1.1.1), the sections (1.1.1) deflect radially and when the needle shield (4) has bypassed the proximal ends of the sections (1.1.1), the sections return to a non-deflected position, wherein, when the needle shield (4) abuts the shoulder sections (1.4), arms (1.3) formed in the body (1.1), the arms (1.3) having distal ends including the shoulder sections (1.4) shaped as portions of a circle arranged in a transverse plane with respect to a longitudinal axis of the carrier (1), deflect until the needle shield (4) bypasses the shoulder sections (1.4) and the collar (1.2) engages the circumferential gap to prevent the syringe (2) from moving in the distal direction (D) relative to the syringe carrier (1).

## Patentansprüche

1. Spritzenträger (1), aufweisend:
eine Spritze (2), die einen Zylinder (2.1) und einen Nadelschutz (4) aufweist, der eine an einem distalen Ende der Spritze (2) angeordnete Nadel (3) abdeckt, wobei ein Umfangsspalt zwischen dem Zylinder (2.1) der Spritze (2) und dem Nadelschutz (4) angeordnet ist, und
einen Körper (1.1), wobei der Zylinder (2.1) in dem Körper (1.1) aufgenommen ist, wobei der Körper (1.1) zumindest zwei Abschnitte (1.1.1) aufweist, die distale Enden mit Schulterabschnitten (1.4) haben, die als Teile eines in einer Querebene bezüglich einer Längsachse des Spritzenträgers (1) angeordneten Kreises ausgebildet sind, wobei die Abschnitte (1.1.1) federnd an einen Bund (1.2) an einem distalen Ende des Körpers (1.1) gekoppelt sind und wobei die Schulterabschnitte (1.4) dazu ausgeführt sind, den Umfangsspalt in Eingriff zu nehmen, um zu verhindern, dass sich die Spritze (2) in eine distale Richtung (D) bezüglich des Spritzenträgers (1) bewegt.

2. Spritzenträger (1) nach Anspruch 1, wobei die Abschnitte (1.1.1) auslenken, wenn sie vom Nadelschutz (4) in Eingriff genommen werden, und zu einer nicht ausgelenkten Position zurückkehren, wenn sie mit dem Nadelschutz (4) außer Eingriff kommen, um einen Fingerflansch (2.3) der Spritze (2) in Eingriff zu nehmen.

3. Spritzenträger (1) nach einem der vorhergehenden Ansprüche, wobei die Schulterabschnitte (1.4) proximal ausgerichtete konturierte Flächen aufweisen, um einen proximalen Teil eines Halses (2.2) der Spritze (2) aufzunehmen, sowie distal ausgerichtete planare Flächen, um an den Nadelschutz (4) anzuschlagen.

4. Spritzenträger (1) nach einem der vorhergehenden Ansprüche, wobei der Körper (1.1) ein oder mehrere Sichtfenster (5) aufweist.

5. Spritzenträger (1) nach Anspruch 4, wobei die Sichtfenster (5) ausgebildet sind, wenn Ausschnitte in den Abschnitten (1.1.1) im Wesentlichen angrenzend sind, wenn die Abschnitte (1.1.1) in der nicht ausgelenkten Position sind.

6. Spritzenträger (1) nach Anspruch 4, wobei um jeden Ausschnitt herum ein Vorsprung (1.6) gebildet ist und die Vorsprünge (1.6) einen Umriss für das Sichtfenster (5) bilden, wenn die Abschnitte (1.1.1) in der nicht ausgelenkten Position sind.

7. Spritzenträger (1) nach einem der vorhergehenden Ansprüche, wobei der Körper (1.1) ein Halterelement (1.7) aufweist, das ausgeführt ist, eine Anschlagfläche bereitzustellen, um zu verhindern, dass die Spritze (2) den Spritzenträger (1) in einer proximalen Richtung (P) löst.

8. Spritzenträger (1) nach einem der vorhergehenden Ansprüche, wobei die beiden federnden Abschnitte (1.1.1), wenn sie zusammen sind, eine zylindrische Form mit einem Innendurchmesser haben, der dem Durchmesser des Zylinders (2.1) entspricht.

9. Spritzenträger (1) nach einem der vorhergehenden Ansprüche, wobei der Bund (1.2) teilweise durch distale Enden der Abschnitte (1.1.1) des Körpers (1.1) gebildet ist, wobei der Bund (1.2) so bemessen ist, dass er ein axiales Einführen der Spritze (2) in den Spritzenträger (1) gestattet.

10. Medikamenten-Verabreichungsvorrichtung, aufweisend den Spritzenträger (1) nach einem der vorhergehenden Ansprüche.

11. Medikamenten-Verabreichungsvorrichtung nach Anspruch 10, wobei die Nadel die Nadel (3) der Spritze (2) ist und wobei die Schulterabschnitte (1.4) den Umfangsspalt in Eingriff nehmen.

12. Medikamenten-Verabreichungsvorrichtung nach Anspruch 11, wobei die Spritze (2) mit einem Medikament vorgefüllt ist.

13. Verfahren zur Montage einer Spritze (2), die einen Zylinder (2.1) und einen eine Nadel (3) der Spritze (2) abdeckenden Nadelschutz (4) aufweist, in einem Spritzenträger (1) nach einem der Ansprüche 1 bis 9, **dadurch gekennzeichnet, dass** das Verfahren Folgendes aufweist: Schieben der Spritze (2) in der distalen Richtung (D) in den Spritzenträger (1), wobei die Abschnitte (1.1.1) radial ausgelenkt werden, wenn der Nadelschutz (4) an proximalen Enden der Abschnitte (1.1.1) anliegt, und die Abschnitte zu einer nicht ausgelenkten Position zurückkehren, wenn der Nadelschutz (4) an den proximalen Enden der Abschnitte (1.1.1) vorbeigegangen ist, wobei, wenn der Nadelschutz (4) an den Schulterabschnitten (1.4) anliegt, in dem Körper (1.1) ausgebildete Arme (1.3), wobei die Arme (1.3) distale Enden mit den Schulterabschnitten (1.4) haben, die als Teile eines in einer Querebene bezüglich einer Längsachse des Trägers (1) angeordneten Kreises ausgebildet sind, so lange ausgelenkt werden, bis der Nadelschutz (4) an den Schulterabschnitten (1.4) vorbeigegangen ist und der Bund (1.2) den Umfangsspalt in Eingriff nimmt, um zu verhindern, dass sich die Spritze (2) in die distale Richtung (D) bezüglich des Spritzenträgers (1) bewegt.

## Revendications

1. Support de seringue (1) comprenant :
une seringue (2) ayant un cylindre (2.1) et un élément de protection d'aiguille (4) recouvrant une aiguille (3) agencé sur une extrémité distale de la seringue (2), un espace circonférentiel étant prévu entre le cylindre (2.1) de la seringue (2) et l'élément de protection d'aiguille (4), et
un corps (1.1), le cylindre (2.1) étant reçu dans le corps (1.1), le corps (1.1) comprenant au moins deux sections (1.1.1) ayant des extrémités distales avec des sections formant épaulements (1.4) formées comme des parties d'un cercle agencé dans un plan transversal par rapport à un axe longitudinal du support de seringue (1), les sections (1.1.1) étant accouplées de manière élastique à un collet (1.2) sur une extrémité distale du corps (1.1), et les sections formant épaulements (1.4) étant adaptées pour interagir avec l'espace circonférentiel pour empêcher la seringue (2) de se déplacer dans une direction distale (D) par rapport au support de seringue (1).

2. Support de seringue (1) selon la revendication 1, les sections (1.1.1) fléchissant lorsqu'elles interagissent avec l'élément de protection d'aiguille (4) et revenant à une position non déformée lorsqu'elles cessent d'interagir avec l'élément de protection d'aiguille (4) pour interagir avec une bride de doigt (2.3) de la seringue (2).

3. Support de seringue (1) selon l'une quelconque des revendications précédentes, les sections formant épaulements (1.4) comprenant des surfaces profilées orientées de façon proximale pour recevoir une partie proximale d'un col (2.2) de la seringue (2) et des surfaces planes orientées de façon distale pour venir en butée contre l'élément de protection d'aiguille (4).

4. Support de seringue (1) selon l'une quelconque des revendications précédentes, le corps (1.1) comprenant une ou plusieurs fenêtres d'observation (5).

5. Support de seringue (1) selon la revendication 4, les fenêtres d'observation (5) étant formées lorsque des découpes dans les sections (1.1.1) sont sensiblement contiguës lorsque les sections (1.1.1) sont en position non fléchie.

6. Support de seringue (1) selon la revendication 4, une saillie (1.6) étant formée autour de chaque découpe, et lorsque les sections (1.1.1) sont dans la position non fléchie, les saillies (1.6) formant un contour pour la fenêtre d'observation (5).

7. Support de seringue (1) selon l'une quelconque des revendications précédentes, le corps (1.1) comprenant un élément de retenue (1.7) adapté pour fournir une surface de butée pour empêcher la seringue (2) de se désassembler du support de seringue (1) dans une direction proximale (P).

8. Support de seringue (1) selon l'une quelconque des revendications précédentes, les deux sections élastiques (1.1.1), lorsqu'elles sont ensemble, ayant une forme cylindrique avec un diamètre interne correspondant au diamètre du cylindre (2.1).

9. Support de seringue (1) selon l'une quelconque des revendications précédentes, le collet (1.2) étant partiellement formé par des extrémités distales des sections (1.1.1) du corps (1.1), le collet (1.2) étant dimensionné pour permettre l'insertion axiale de la seringue (2) dans le support de seringue (1).

10. Dispositif d'administration de médicament comprenant le support de seringue (1) selon l'une quelconque des revendications précédentes.

11. Dispositif d'administration de médicament selon la revendication 10, l'aiguille étant l'aiguille (3) de la seringue (2), et les sections formant épaulements (1.4) interagissant avec l'espace circonférentiel.

12. Dispositif d'administration de médicament selon la revendication 11, la seringue (2) étant pré-remplie d'un médicament.

13. Procédé d'assemblage d'une seringue (2) ayant un cylindre (2.1) et un élément de protection d'aiguille (4) recouvrant une aiguille (3) de la seringue (2) dans un support de seringue (1) selon l'une quelconque des revendications 1 à 9, **caractérisé en ce que** le procédé comprend : le glissement de la seringue (2) dans la direction distale (D) dans le support de seringue (1), lorsque l'élément de protection d'aiguille (4) vient en butée contre les extrémités proximales des sections (1.1.1), les sections (1.1.1) fléchissant radialement et lorsque l'élément de protection d'aiguille (4) a contourné les extrémités proximales des sections (1.1.1), les sections revenant à une position non fléchie, lorsque l'élément de protection d'aiguille (4) vient en butée contre les sections formant épaulements (1.4), des bras (1.3) formés dans le corps (1.1), les bras (1.3) ayant des extrémités distales comprenant les sections formant épaulements (1.4) formées comme des parties d'un cercle agencé dans un plan transversal par rapport à un axe longitudinal du support (1), se déformant jusqu'à ce que l'élément de protection d'aiguille (4) contourne les sections formant épaulements (1.4) et que le collet (1.2) s'engage dans l'espace circonférentiel pour empêcher la seringue (2) de se déplacer dans la direction distale (D) par rapport au support de seringue (1).
